# EUROPEAN PATENT APPLICATION

(11) **EP 4 285 855 A1**
(43) Date of publication of application: **06.12.2023**
(21) Application number: 22745608.4
(22) Date of filing: 14.01.2022
(51) Int. Cl.: A61B 34/20, A61B 3/13, A61F 9/007

(54) **IMAGE PROCESSING DEVICE, IMAGE PROCESSING METHOD, AND SURGICAL MICROSCOPE SYSTEM**

(30) Priority: 29.01.2021 JP 2021013119; 11.06.2021 JP 2021098077
(71) Applicant: Sony Group Corporation, Tokyo 108-0075 (JP); Sony Olympus Medical Solutions Inc., Hachioji-shi, Tokyo 192-0904 (JP)
(72) Inventor: OOTSUKI, Tomoyuki, Tokyo 108-0075 (JP); ENOKI, Junichiro, Tokyo 108-0075 (JP); USHIRODA, Hiroshi, Hachioji-shi, Tokyo 192-0904 (JP); FUKAYA, Koji, Hachioji-shi, Tokyo 192-0904 (JP); SUGIE, Yuki, Tokyo 108-0075 (JP); KASHIMA, Koji, Tokyo 108-0075 (JP); HOSOI, Izumu, Tokyo 108-0075 (JP)
(74) Representative: D Young & Co LLP
(86) International application number: PCT/JP2022/001074
(87) International publication number: WO 2022/163383

(57) **Abstract**

An image processing device (13) according to one aspect of the present disclosure includes: an image input unit (13a) that receives a surgical field image with respect to a patient's eye; an eyeball tracking unit (13b) as an example of a part detection unit that detects a specific part of the eye in the surgical field image; and a display image generation unit (13c) that generates a display image including a first position indicator indicating a position a predetermined distance away in a specific direction from the specific part detected by the eyeball tracking unit (13b).

## Description

### Field

The present disclosure relates to an image processing device, an image processing method, and a surgical microscope system.

### Background

During ophthalmic surgery, incision or puncturing needs to be performed on an eyeball based on the anatomical features of the eyeball. For this reason, an increase in complexity tends to arise in the work process, such as referring to a preoperative plan and measuring the distance from a specific part. Specifically, for example, in installing a port for inserting an instrument or the like into an eyeball in vitreous surgery, in order to install the port at the position of the ciliary pars plana while avoiding blood vessels, the port needs to be installed a certain distance away from the corneal limbus to the outside while avoiding the orientation corresponding to a specific preoperative direction (for example, clock positions of 3 o'clock and 9 o'clock directions). Therefore, it is common to mark information on the distance from the corneal limbus on the eyeball using an instrument such as a caliper. It should be noted that Patent Document 1 proposes a technique for changing the position of a preoperative plan-indicating mark in accordance with the result of eyeball tracking.

### Citation List

### Patent Literature

Patent Literature 1: JP 2011-200667 A

### Summary

### Technical Problem

However, in a case where the information on the distance from the corneal limbus is marked on the eyeball as described above, an operator needs to perform measurement on his or her own using an instrument such as a caliper. For this reason, an ophthalmic surgery-related work process is complicated for the operator.

In this regard, the present disclosure proposes an image processing device, an image processing method, and a surgical microscope system enabling ophthalmic surgery-related work process simplification.

### Solution to Problem

An image processing device according to the embodiment of the present disclosure includes: an image input unit that receives a surgical field image with respect to a patient's eye; a part detection unit that detects a specific part of the eye in the surgical field image; and a display image generation unit that generates a display image including a first position indicator indicating a position a predetermined distance away in a specific direction from the specific part detected by the part detection unit.

An image processing method according to the embodiment of the present disclosure includes: by an image processing device, receiving a surgical field image with respect to a patient's eye; detecting a specific part of the eye in the surgical field image; and generating a display image including a first position indicator indicating a position a predetermined distance away in a specific direction from the specific part.

A surgical microscope system according to the embodiment of the present disclosure includes: a surgical microscope that obtains a surgical field image with respect to a patient's eye; an image processing device that generates a display image; and a display device that displays the display image, wherein the image processing device includes an image input unit that receives the surgical field image, a part detection unit that detects a specific part of the eye in the surgical field image, and a display image generation unit that generates the display image including a first position indicator indicating a position a predetermined distance away in a specific direction from the specific part detected by the part detection unit.

### Brief Description of Drawings

FIG. 1 is a diagram illustrating an example of a schematic configuration of a surgical microscope system according to a first embodiment.
FIG. 2 is a diagram illustrating an example of a schematic configuration of a surgical microscope according to the first embodiment.
FIG. 3 is a diagram illustrating an example of a schematic configuration of an image processing device according to the first embodiment.
FIG. 4 is a diagram illustrating Example 1 of a display image according to the first embodiment.
FIG. 5 is a diagram illustrating an example of a schematic configuration of an image processing device according to a second embodiment.
FIG. 6 is a diagram illustrating Example 2 of a display image according to the second embodiment.
FIG. 7 is a diagram illustrating Example 3 of the display image according to the second embodiment.
FIG. 8 is a diagram illustrating Example 4 of the display image according to the second embodiment.
FIG. 9 is a diagram illustrating Example 5 of the display image according to the second embodiment.
FIG. 10 is a diagram illustrating Example 6 of the display image according to the second embodiment.
FIG. 11 is a diagram illustrating Example 7 of the display image according to the second embodiment.
FIG. 12 is a diagram illustrating Example 8 of the display image according to the second embodiment.
FIG. 13 is a diagram illustrating Example 9 of the display image according to the second embodiment.
FIG. 14 is a diagram illustrating Example 10 of a display image according to a third embodiment.
FIG. 15 is a diagram illustrating Example 11 of the display image according to the third embodiment.
FIG. 16 is a diagram illustrating Example 12 of the display image according to the third embodiment.
FIG. 17 is a diagram illustrating Example 13 of the display image according to the third embodiment.
FIG. 18 is a diagram illustrating Example 14 of the display image according to the third embodiment.
FIG. 19 is a diagram illustrating Example 15 of the display image according to the third embodiment.
FIG. 20 is a diagram illustrating Example 16 of the display image according to the third embodiment.
FIG. 21 is a diagram illustrating Example 17 of the display image according to the third embodiment.
FIG. 22 is a diagram illustrating Example 18 of the display image according to the third embodiment.
FIG. 23 is a diagram illustrating an example of a schematic configuration of a computer according to each embodiment of the present disclosure.

### Description of Embodiments

Hereinafter, an embodiment of the present disclosure will be described in detail with reference to the drawings. It should be noted that the device, method, system, and the like according to the present disclosure are not limited by this embodiment. In addition, in each of the following embodiments, basically the same parts are denoted by the same reference numerals with redundant description omitted.

Each of the one or more embodiments (including examples and modification examples) described below can be implemented independently. Meanwhile, at least some of the embodiments described below may be implemented in combination with at least some of the other embodiments as appropriate. These embodiments may include mutually different novel features. Therefore, these plurality of embodiments are capable of contributing to solving mutually different purposes or problems and having mutually different effects.

The present disclosure will be described in the following item order.
1. First Embodiment
   1-1. Example of Schematic Configuration of Surgical Microscope System
   1-2. Example of Schematic Configuration of Surgical Microscope
   1-3. Example of Schematic Configuration of Image Processing Device and Image Processing
   1-4. Action and Effect
2. Second Embodiment
   2-1. Example of Schematic Configuration of Image Processing Device and Image Processing
   2-2. Action and Effect
   2-3. Example of Fixed Pattern Display Function
3. Third Embodiment
   3-1. Example of Boundary Display Function Based on Difference in Display Form
   3-2. Action and Effect
4. Example of Schematic Configuration of Computer
5. Note

### <1. First Embodiment>

### <1-1. Example of Schematic Configuration of Surgical Microscope System>

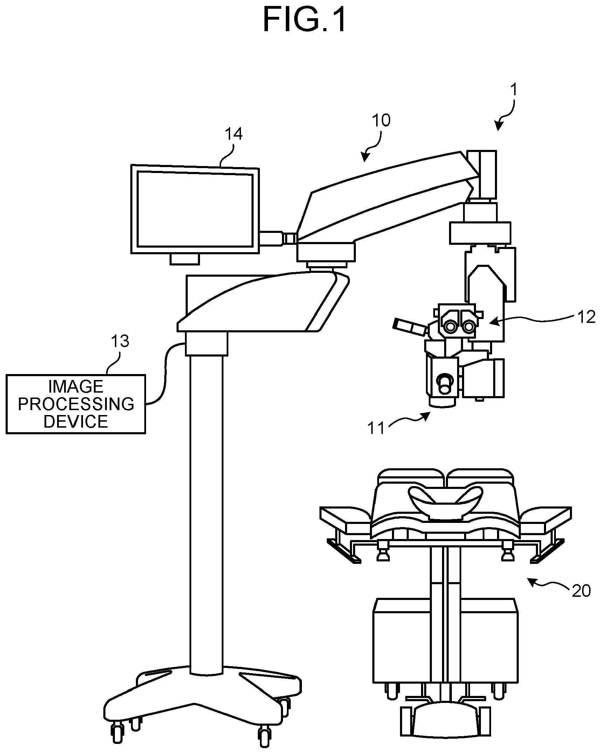

An example of a schematic configuration of a surgical microscope system 1 according to a first embodiment will be described with reference to FIG. 1. FIG. 1 is a diagram illustrating the example of the schematic configuration of the surgical microscope system 1 according to the first embodiment.

As illustrated in FIG. 1, the surgical microscope system 1 has a surgical microscope 10 and a patient bed 20. This surgical microscope system 1 is a system used for eye surgery. A patient undergoes eye surgery while lying on the patient bed 20. In addition, an operator as a doctor performs the surgery while observing the patient's eye with the surgical microscope 10.

The surgical microscope 10 has an objective lens 11, an eyepiece lens 12, an image processing device 13, and a monitor 14.

The objective lens 11 and the eyepiece lens 12 are lenses for magnifying and observing the eye of the patient as a surgery target.

The image processing device 13 performs predetermined image processing on an image captured via the objective lens 11 to output various images, various types of information, and the like.

The monitor 14 displays the image captured via the objective lens 11, the various images and various types of information generated by the image processing device 13, and the like. This monitor 14 may be provided separately from the surgical microscope 10.

In this surgical microscope system 1, the operator performs the surgery while, for example, looking through the eyepiece lens 12 and observing the patient's eye via the objective lens 11. In addition, the operator performs the surgery while checking, for example, the various images (for example, pre- and post-image processing images) and various types of information displayed on the monitor 14.

### <1-2. Example of Schematic Configuration of Surgical Microscope>

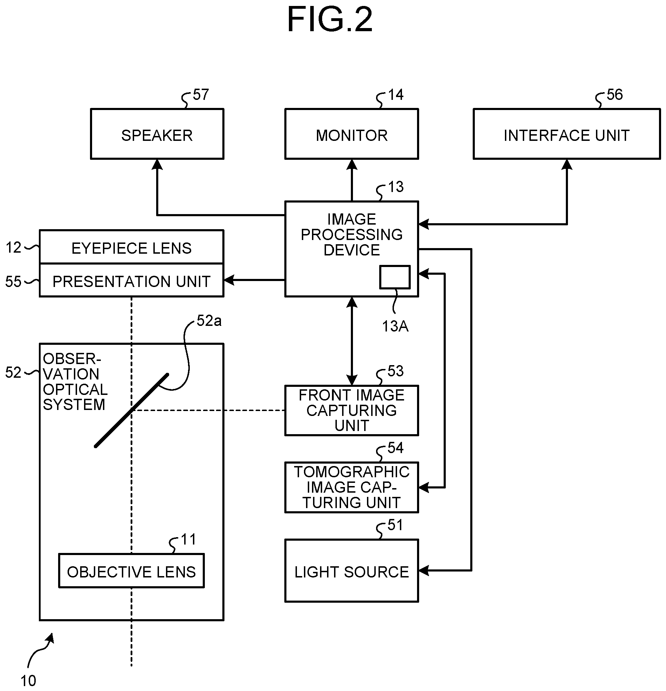

An example of a schematic configuration of the surgical microscope 10 according to the first embodiment will be described with reference to FIG. 2. FIG. 2 is a diagram illustrating the example of the schematic configuration of the surgical microscope 10 according to the first embodiment.

As illustrated in FIG. 2, the surgical microscope 10 has, in addition to the objective lens 11, the eyepiece lens 12, the image processing device 13, and the monitor 14, a light source 51, an observation optical system 52, a front image capturing unit 53, a tomographic image capturing unit 54, a presentation unit 55, an interface unit 56, and a speaker 57. It should be noted that the monitor 14 and the presentation unit 55 correspond to display devices.

The light source 51 emits illumination light under the control of a control unit 13A of the image processing device 13 to illuminate the patient's eye.

The observation optical system 52 is configured from, for example, optical elements such as the objective lens 11, a half mirror 52a, and a lens (not illustrated). This observation optical system 52 guides light (observation light) reflected from the patient's eye to the eyepiece lens 12 and the front image capturing unit 53.

Specifically, the light reflected from the patient's eye enters the half mirror 52a as observation light via the objective lens 11, the lens (not illustrated), or the like. Approximately half of the observation light incident on the half mirror 52a passes through the half mirror 52a as it is and enters the eyepiece lens 12 via the transmissive presentation unit 55. Meanwhile, the other half of the observation light incident on the half mirror 52a is reflected by the half mirror 52a and enters the front image capturing unit 53.

The front image capturing unit 53 is configured by, for example, a video camera. This front image capturing unit 53 receives the observation light incident from the observation optical system 52 and performs photoelectric conversion to capture a front image as an image in which the patient's eye is observed from the front, that is, an image in which the patient's eye is photographed from an approximate eye axis direction. The front image capturing unit 53 captures (images) the front image under the control of the image processing device 13 and supplies the obtained front image to the image processing device 13.

The tomographic image capturing unit 54 is configured by, for example, optical coherence tomography (OCT), a Scheimpflug camera, or the like. This tomographic image capturing unit 54 captures (images) a tomographic image as a cross-sectional image of the patient's eye under the control of the image processing device 13 and supplies the obtained tomographic image to the image processing device 13. Here, the tomographic image is an image of a cross section of the patient's eye in a direction substantially parallel to the eye axis direction.

It should be noted that although the tomographic image capturing unit 54 acquires the tomographic image by, for example, using infrared light based on the principle of interference, the optical path of the infrared light at that time and a part of the optical path of the observation light in the observation optical system 52 may be a common optical path.

The eyepiece lens 12 collects the observation light incident from the observation optical system 52 via the presentation unit 55 to form an optical image of the patient's eye. As a result, the optical image of the patient's eye is observed by the operator looking through the eyepiece lens 12.

The presentation unit 55 is configured by a transmissive display device or the like and is disposed between the eyepiece lens 12 and the observation optical system 52. This presentation unit 55 transmits the observation light incident from the observation optical system 52 to cause the light to enter the eyepiece lens 12 and, as needed, presents (displays) the various images (for example, the front and tomographic images) and various types of information supplied from the image processing device 13. The various images, various types of information, and the like may be, for example, superimposed on the optical image of the patient's eye and presented or presented in the peripheral portion of the optical image so as not to disturb the optical image.

The image processing device 13 has the control unit 13A, which controls the operation of the surgical microscope 10 as a whole. For example, the control unit 13A changes the illumination conditions of the light source 51 or changes the zoom magnification of the observation optical system 52. In addition, the control unit 13A controls the image acquisition of the front image capturing unit 53 and the tomographic image capturing unit 54 based on, for example, operation information of the operator or the like supplied from the interface unit 56.

The interface unit 56 is configured by, for example, a communication unit. The communication unit receives a command from an operation unit such as a touch panel superimposed on the monitor 14, a controller, and a remote controller (not illustrated) or communicates with an external device. This interface unit 56 supplies the image processing device 13 with, for example, information corresponding to the operation of the operator or the like. In addition, the interface unit 56 outputs, for example, device control information for external device control supplied from the image processing device 13 to an external device.

The monitor 14 displays various images such as front and stereo images and various types of information under the control of the control unit 13A of the image processing device 13.

In a case where, for example, a dangerous situation is detected during the surgery, the speaker 57 outputs a sound such as buzzer and melody sounds, a message (voice), and the like under the control of the control unit 13A of the image processing device 13 in order to notify, for example, the operator of the dangerous situation. It should be noted that the surgical microscope 10 may be provided with a rotating light or an indicator light (lamp) for notifying, for example, the operator of the dangerous situation.

In the surgical microscope system 1 configured as described above, the operator does not have to perform distance measurement or the like and work process simplification can be achieved by detecting a specific part of the eye using image recognition with respect to a surgical field image and marking a position a predetermined distance away in a specific direction from the specific part of the eyeball, for example, superimposing a mark as a treatment indicator at, for example, a part a certain distance away from the specific part. The mark functions as a position indicator.

### <1-3. Example of Schematic Configuration of Image Processing Device and Image Processing>

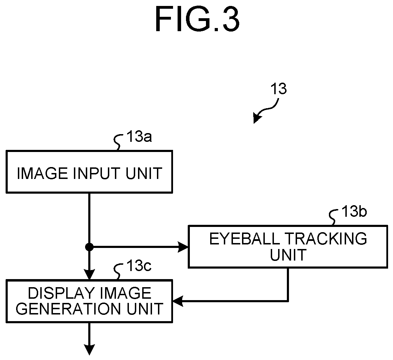

An example of a schematic configuration of the image processing device 13 and image processing according to the first embodiment will be described with reference to FIG. 3. FIG. 3 is a diagram illustrating the example of the schematic configuration (configuration and processing flow) of the image processing device 13 according to the first embodiment.

As illustrated in FIG. 3, the image processing device 13 includes an image input unit 13a, an eyeball tracking unit 13b, and a display image generation unit 13c.

The image input unit 13a receives a surgical field image (front image) from the front image capturing unit 53 (see FIG. 2) and supplies the received surgical field image (for example, surgical field image at surgery initiation or real-time surgical field image during surgery) to the eyeball tracking unit 13b, the display image generation unit 13c, and the like.

The eyeball tracking unit 13b tracks the eyeball in a real-time surgical field image by, for example, comparing a surgical field image at surgery initiation with the real-time surgical field image. This eyeball tracking unit 13b detects the posture and position of the eyeball in the real-time surgical field image and a specific part of the eye (for example, corneal limbus) by tracking the eyeball and supplies the display image generation unit 13c with tracking result information related to the detection result (for example, eyeball posture and position and corneal limbus position). The eyeball posture information as part of the detection result includes position information related to the orientation of the eyeball around the eye axis (position in the direction of rotation around the eye axis). The eyeball tracking unit 13b corresponds to a part detection unit that detects a specific part of the eye.

Based on the tracking result information supplied from the eyeball tracking unit 13b, the display image generation unit 13c generates a display image including a mark indicating a position a predetermined distance away in a specific direction from a specific part of the eyeball. For example, installation information related to a specific direction and a predetermined distance for port installation is set in advance. The display image generation unit 13c has the preset installation information and, based on the installation information, sets a mark at a position a predetermined distance away in a specific direction from the position of the specific part based on the tracking result information. It should be noted that in a case where the mark needs to be changed in accordance with a change in eyeball posture or position, the display image generation unit 13c generates the display image while changing the posture or position of the mark in accordance with the change in eyeball posture or position based on the eyeball posture and position information included in the tracking result information.

An example will be described in which the image processing device 13 configured as described above fulfills a guidance function to assist the operator in ophthalmic surgery, for example, a guidance function to guide the position of port installation in vitreous surgery. Specifically, the port installation position is guided by a mark.

Here, in general, a port as a surgical instrument is provided in the eye in performing vitreous surgery. Specifically, although it is common to provide three ports in total (ports for perfusate supply, illumination insertion, and surgical tool illumination), approximately one or two ports may be added for, for example, chandelier lighting that illuminates the whole. Although these ports are provided in the pars plana ciliaris without exception, in a case where this pars plana ciliaris is viewed from the outside of the eye, the port is often installed at a position approximately 4 mm away from the corneal limbus although there are individual differences. Therefore, in general, the position of approximately 4 mm is measured with a caliper or the like and the port is installed at that position. It should be noted that approximately 4 mm is a statistical distance from the corneal limbus to the port installation position.

However, blood vessels (for example, long posterior ciliary artery) are near the 3 o'clock direction and the 9 o'clock direction (hereinafter, simply referred to as 3 o'clock and 9 o'clock directions) of the clock position when viewed from the angle of the eye at the preoperative sitting position, and thus the port is installed with that part avoided so that injury or bleeding is prevented. Although the above 3 o'clock and 9 o'clock directions are slightly rotated with respect to the sitting position due to the supine position during the surgery and thus it is necessary to take this into consideration, it is common to perform the installation by selecting a position slightly away from the 3 o'clock and 9 o'clock directions during the surgery.

In the present embodiment, guidance is performed as follows for the purpose of assisting in such port installation or the like. Based on the image from the image input unit 13a, the eyeball tracking unit 13b detects the position of the corneal limbus in the eyeball and supplies information on the detection result to the display image generation unit 13c. In the display image generation unit 13c, based on the corneal limbus detection result from the eyeball tracking unit 13b on the image from the image input unit 13a, a guidance mark is drawn at a position a certain predetermined distance (for example, approximately 4 mm) away from the corneal limbus in the specific direction toward each treatment target point from the center of the corneal limbus as an example of the specific part. As a result of such guidance, the operator does not have to perform positioning on his or her own using a caliper or the like with reference to the corneal limbus, which results in a decrease in surgical complexity. Various images can be used as the display image for guidance.

### (Example 1 of Display Image)

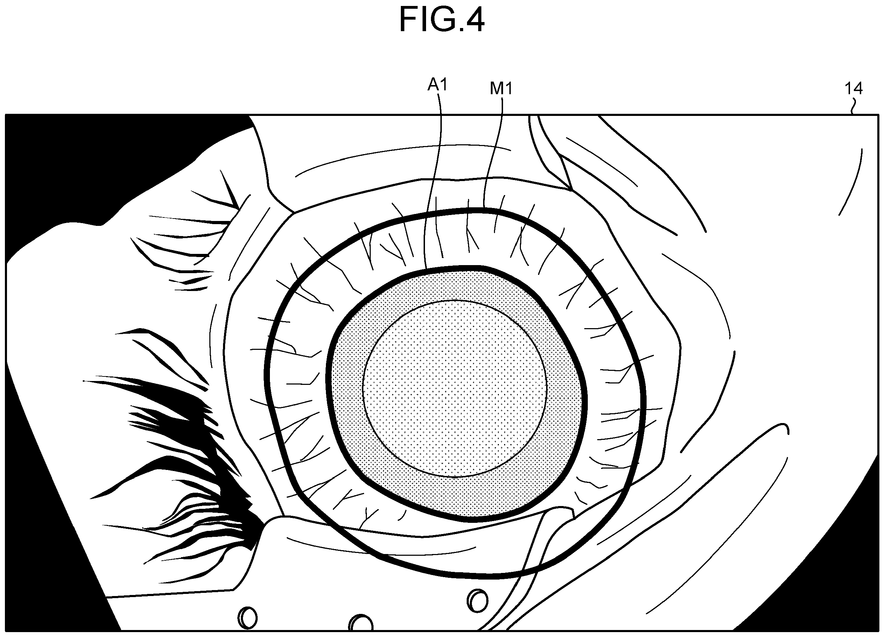

FIG. 4 is a diagram illustrating Example 1 of the display image according to the first embodiment. As illustrated in FIG. 4, the corneal limbus detection result, that is, a corneal limbus-indicating mark A1 is presented and, further, an annular mark M1 is presented at a position a predetermined distance away from the corneal limbus. These marks A1 and M1 are superimposed on the real-time surgical field image to generate the display image. This display image is displayed by both or one of the monitor 14 and the presentation unit 55. It should be noted that the mark M1 indicates a position a certain predetermined distance (for example, approximately 4 mm) away from the corneal limbus in the specific direction toward each treatment target point from the center of the corneal limbus. The mark M1 corresponds to a first position indicator. In addition, the mark A1 corresponds to a second position indicator.

Such a display image is visually recognized by the operator, and the operator can grasp the treatment target position. Specifically, a specific part of the eyeball can be detected by using the eyeball tracking unit 13b with respect to the surgical field image and, based on the detected information, the display image generation unit 13c is capable of marking a position a predetermined distance away from the specific part in the specific direction. As a result, it is possible to determine the position of the treatment target. The operator can avoid the complexity of performing measurement on his or her own using an instrument such as a caliper. In addition, detailed guidance that cannot be performed only from the front image of the eyeball is possible.

In addition, even in a case where the position of the detection result deviates from the actual limbus position, by presenting the corneal limbus detection result, that is, the corneal limbus-indicating mark A1, the operator can grasp the deviation and reflect the deviation in the treatment target position such as the port installation position.

### <1-4. Action and Effect>

As described above, according to the first embodiment, the image input unit 13a receives a surgical field image with respect to a patient's eye, the eyeball tracking unit 13b as an example of the part detection unit detects a specific part of the eye in the surgical field image, and the display image generation unit 13c generates a display image including the first position indicator (such as the mark M1) indicating a position a predetermined distance away from the specific part in a specific direction. As a result, an operator can grasp the position the predetermined distance away in the specific direction from the specific part of the eye, that is, a treatment target position. Therefore, the operator does not have to perform measurement on his or her own using an instrument such as a caliper in order to grasp the treatment target position, and thus an ophthalmic surgery-related work process can be simplified.

In addition, the first position indicator may be, for example, a curved line or an annular line indicating a position a predetermined distance away from a specific part or a line segment substantially matching a part of the annular line. As a result, it is possible to present a plurality of treatment target positions (for example, three port installation positions) on the curved line, annular line or the line segment, and thus the operator can easily grasp the treatment target position.

In addition, the display image generation unit 13c generates the second position indicator (such as the mark A1) different from the first position indicator related to the treatment target position and generates a display image including the first position indicator and the second position indicator. As a result, the operator can grasp another position to be intraoperatively recognized while grasping the treatment target position. The mark A1, which is an example of the second position indicator, is a mark indicating the corneal limbus, which is a specific part.

### <2. Second Embodiment>

### <2-1. Example of Schematic Configuration of Image Processing Device and Image Processing>

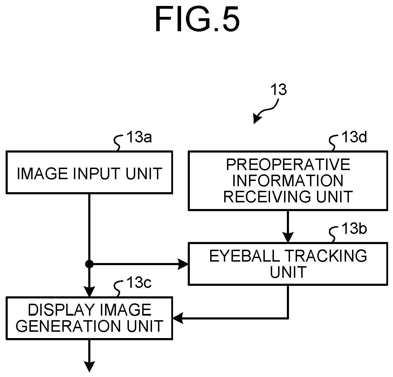

An example of a schematic configuration of an image processing device 13 and image processing according to a second embodiment will be described with reference to FIG. 5. FIG. 5 is a diagram illustrating the example of the schematic configuration (configuration and processing flow) of the image processing device 13 according to the second embodiment.

As illustrated in FIG. 5, the image processing device 13 includes a preoperative information receiving unit 13d in addition to the units 13a to 13c according to the first embodiment.

The preoperative information receiving unit 13d receives preoperative information based on a preoperative plan with respect to the patient's eye and supplies the received preoperative information to the eyeball tracking unit 13b. The preoperative information includes, for example, information indicating the separation distance between a specific part (for example, corneal limbus) and a treatment target point (treatment target position), information related to a specific part (for example, blood vessel running portion in the eye), and information indicating a predetermined direction. The specific part-related information includes, for example, information related to the position, range, and the like of the specific part. In addition, the predetermined direction is, for example, the left-right direction (3 o'clock and 9 o'clock directions) orthogonal to the eye axis of a preoperative image of the eye or a direction predetermined by the operator.

The eyeball tracking unit 13b supplies the display image generation unit 13c with the preoperative information supplied from the preoperative information receiving unit 13d in addition to the tracking result information according to the first embodiment. It should be noted that as in the first embodiment, the eyeball tracking unit 13b supplies the display image generation unit 13c with the posture and position of the eyeball in the real-time surgical field image and the tracking result information related to the specific part of the eye.

The display image generation unit 13c generates a display image including a treatment target point-related mark and a preoperative information-related mark based on, for example, the tracking result information and the preoperative information supplied from the eyeball tracking unit 13b. For example, the display image generation unit 13c generates a mark indicating the blood vessel running portion in the eye, a mark extending in the left-right direction orthogonal to the eye axis of the preoperative image of the eye, a mark extending in a direction predetermined by the operator, or the like as the preoperative information-related mark.

It should be noted that the preoperative information-related mark is a mark that has a posture related to the orientation of the eyeball around the eye axis (position in the direction of rotation around the eye axis). Therefore, the display image generation unit 13c generates a display image while changing the position of the mark in accordance with a change in eyeball position and, in addition, changing the posture of the mark in accordance with a change in the eyeball posture based on the eyeball posture information included in the tracking result information. For example, the display image generation unit 13c adjusts the posture (orientation) of the mark to match the amount and direction of change in eyeball posture (orientation) and changes the posture of the mark so as to eliminate a change in mark posture with respect to the eyeball.

Here, if the distribution range of the pars plana ciliaris is preoperatively measured and the measured distribution range of the pars plana ciliaris is input to the image processing device 13 as preoperative information, it is possible to perform appropriate port installation with individual differences taken into consideration and without relying on the statistical (average) separation distance between the corneal limbus and the treatment target point. Alignment of the pars plana ciliaris between a preoperative image and an intraoperative surgical field image can be achieved by preoperatively grasping the positional relationship between the preoperative image and the pars plana ciliaris. Usable for the alignment is, for example, preoperative and intraoperative blood vessel pattern matching and iris pattern matching. In a case where a preoperative blood vessel running examination result is usable, more accurate treatment can be achieved than with the preoperative 3 o'clock and 9 o'clock directions, which are statistical positions.

### (Example 2 of Display Image)

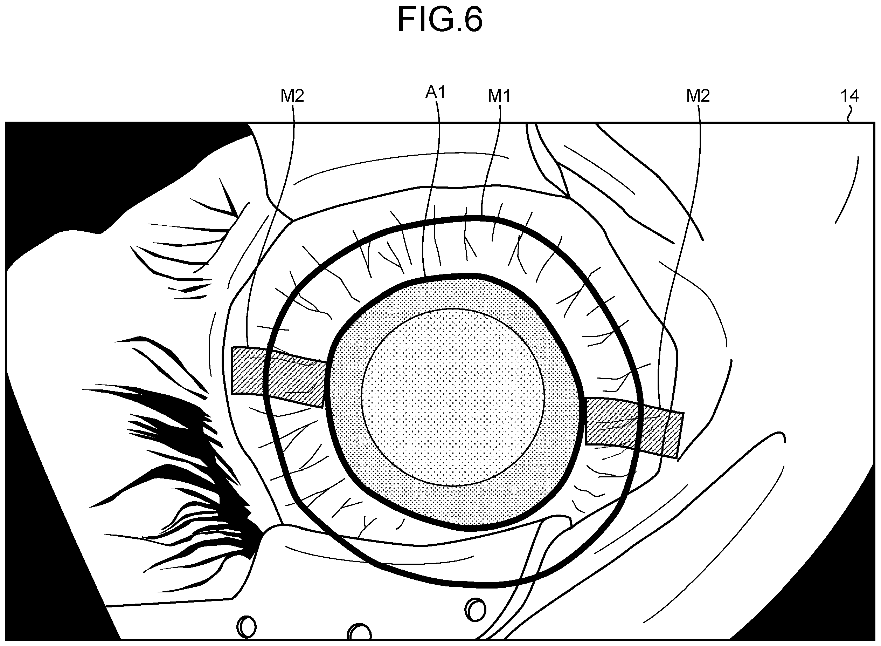

FIG. 6 is a diagram illustrating Example 2 of the display image according to the second embodiment. As illustrated in FIG. 6, a mark M2 indicating a blood vessel running portion is presented in addition to the mark A1 and the mark M1 illustrated in FIG. 5. The mark M2 is a line with a constant width and indicates the position and range of the blood vessel running portion. The mark M2 corresponds to the second position indicator.

The mark M1 is generated in accordance with the separation distance between the corneal limbus and the treatment target point based on preoperative information. By using a preoperative pars plana ciliaris measurement result as the preoperative information, it is possible to draw the mark M1 for port installation position guidance without using a statistical distance from the corneal limbus.

The mark M2 is generated in accordance with the blood vessel running portion based on the preoperative information. Port installation at the blood vessel position leads to blood vessel injury or bleeding and thus should be avoided. Therefore, by presenting the mark M2, the doctor can grasp the position of the blood vessel running portion in detail, and thus the port can be installed with low risk at a position near the blood vessel.

### (Example 3 of Display Image)

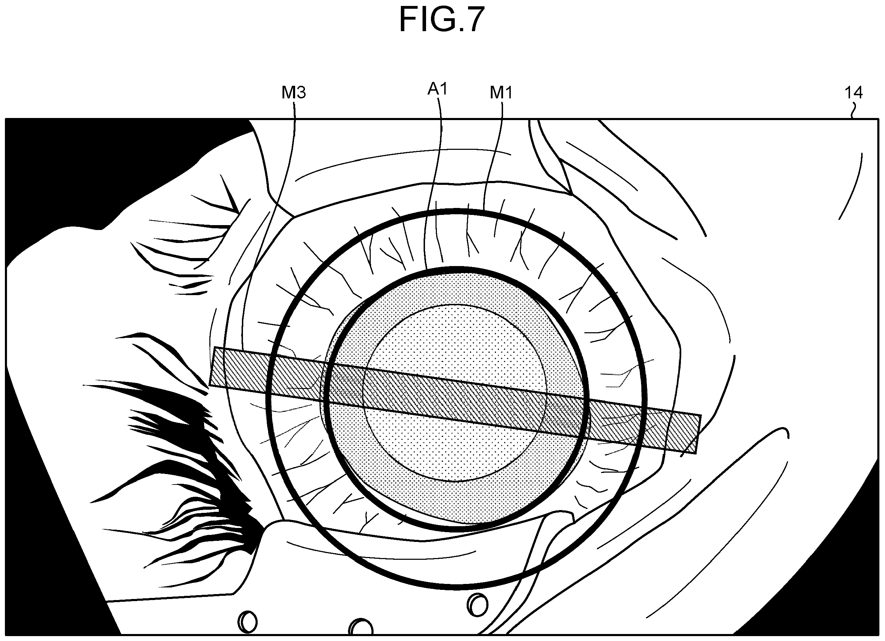

FIG. 7 is a diagram illustrating Example 3 of the display image according to the second embodiment. As illustrated in FIG. 7, each of the marks A1 and M1 is presented as a perfect circle. Further, a mark M3 indicating the 3 o'clock and 9 o'clock directions based on preoperative information is presented. The mark M3 is, for example, a line with a constant width, and the eye is overlaid with the mark M3. The orientation of the mark M3 (position in the direction of rotation around the eye axis) may be changed in accordance with the movement (orientation) of the eyeball so as to be the same at surgery initiation and in real time. It should be noted that the mark M3 may be translucent so that the image directly below the mark M3 can be visually recognized. The mark M3 corresponds to the second position indicator.

A perfect circle, an ellipse, or a part thereof (for example, circular arc) can be used as the mark M1. In addition, the corneal limbus as an example of the specific part may be modeled in a simple shape such as a perfect circle and an ellipse. At this time, a perfect circle, an ellipse, or a part thereof (for example, circular arc) can be used as the mark A1 as with the mark M1.

Here, statistically, most blood vessels run in the preoperative 3 o'clock and 9 o'clock directions. The operator visually recognizes the mark M3 indicating the 3 o'clock and 9 o'clock directions based on preoperative information and can grasp the intraoperative blood vessel position and range in the surgical field image.

In addition, in trying to detect the specific part with high precision, the precision increases if the detection is successful, and yet the risk of significant misdetection as a whole may increase. At this time, a significant deviation (poor detection) can be suppressed by using a simple shape (shape model) such as a perfect circle.

### (Example 4 of Display Image)

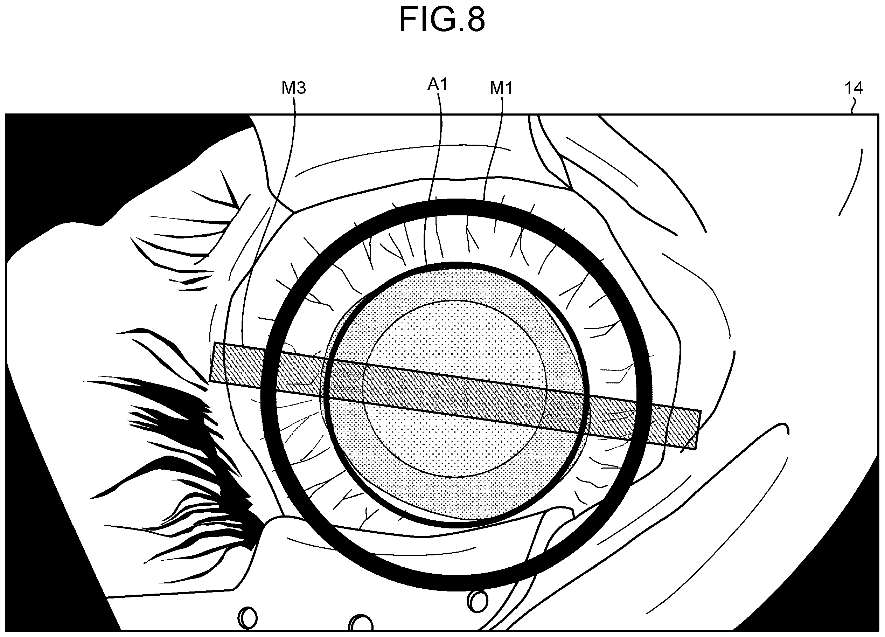

FIG. 8 is a diagram illustrating Example 4 of the display image according to the second embodiment. As illustrated in FIG. 8, the perfect circle mark M1 is a line with a constant width wider than that illustrated in FIG. 7. For example, the position where puncturing into the pars plana ciliaris is possible has a constant width in terms of distance. In other words, by giving the mark M1 a certain width and presenting the mark M1 with the constant width, the operator can easily understand the position and range where puncturing into the pars plana ciliaris is possible.

### (Example 5 of Display Image)

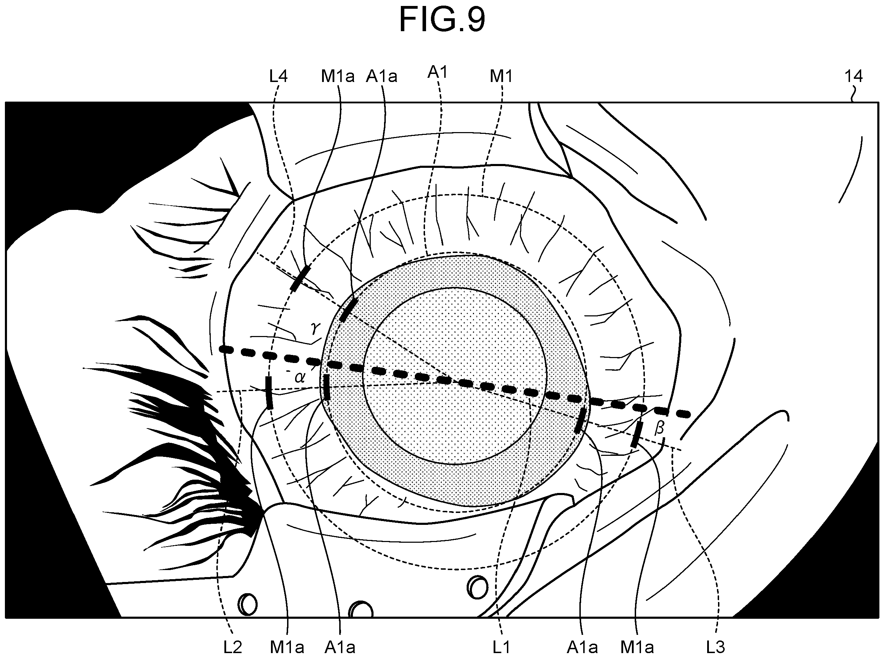

FIG. 9 is a diagram illustrating Example 5 of the display image according to the second embodiment. As illustrated in FIG. 9, the perfect circle mark A1 is partially presented as a plurality of partial marks A1a. Likewise, the perfect circle mark M1 is partially presented as a plurality of partial marks M1a. Each partial mark A1a and each partial mark M1a are disposed at angles α, β, and y. The partial mark M1a corresponds to the first position indicator.

In the example of FIG. 9, a straight line L1 extending in the direction of extension of the mark M3 illustrated in FIG. 7 is used as a reference, and a straight line L2 has the angle α, a straight line L3 has the angle β, and a straight line L4 has the angle y with respect to the straight line L1. The partial marks A1a are presented at the intersection of the perfect circle mark A1 and the straight line L2, the intersection of the perfect circle mark A1 and the straight line L3, and the intersection of the perfect circle mark A1 and the straight line L4. In addition, the partial marks M1a are presented at the intersection of the perfect circle mark M1 and the straight line L2, the intersection of the perfect circle mark M1 and the straight line L3, and the intersection of the perfect circle mark M1 and the straight line L4. It should be noted that the straight lines L1 to L4 may be presented with or without exception.

Operators often use the same port installation direction to meet their preferences. Therefore, in the manner described above, treatment following an operator's routine can be appropriately guided. Since unnecessary marks are reduced, the surgical field is easy to see, the positional deviation of the limbus and the detection result thereof is easy to observe, and it is easy to perform treatment at a position reflecting the deviation with respect to a port installation mark. In addition, puncturing into a blood vessel position can be prevented by an angle such as α, β, and y in FIG. 9 having a certain value or more.

In addition, although a direction based on preoperative information such as the mark M3 is used above as a reference direction for giving an angle such as α, β, and y, the operator's preference may be reflected with respect to the eye posture at surgery initiation or the like. For example, a port installation guidance mark may be given using, as a reference, an angle direction at surgery initiation or the like determined in advance in accordance with the operator's preference as well as the blood vessel running direction or the 3 o'clock and 9 o'clock directions as a reference direction for port installation position setting during vitreous surgery. As a result, easy-to-see marks can be presented for individual operators.

### (Example 6 of Display Image)

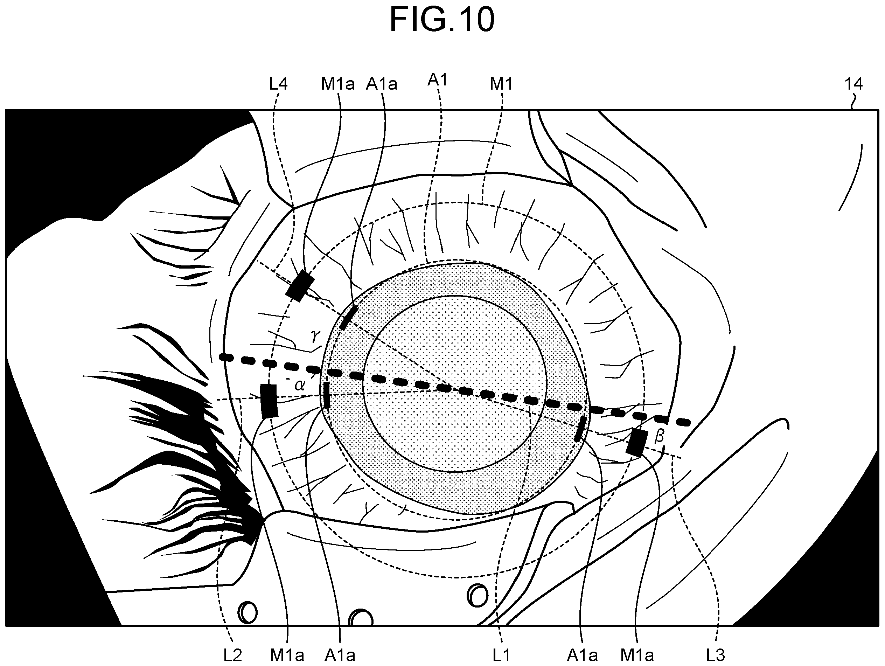

FIG. 10 is a diagram illustrating Example 6 of the display image according to the second embodiment. As illustrated in FIG. 10, the partial mark M1a is a line with a constant width wider than that illustrated in FIG. 9. In this manner, a width may be given to the partial mark M1a, which is a mark for port installation position guidance. As a result, the range of the port installation position is easy to understand.

### (Example 7 of Display Image)

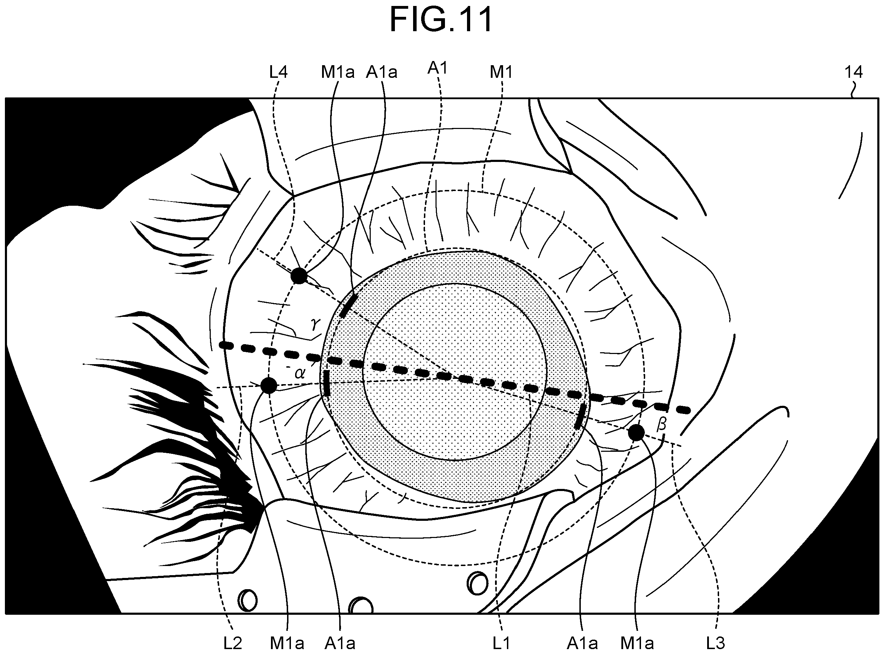

FIG. 11 is a diagram illustrating Example 7 of the display image according to the second embodiment. As illustrated in FIG. 11, the partial mark M1a is formed in a circular shape larger in width (diameter) than that illustrated in FIG. 9. In this manner, as the shape of the partial mark M1a, a figure such as a circle, a polygon, and a star shape may be used as a shape other than a line with a width. As a result, the range of the port installation position is easy to understand.

### (Example 8 of Display Image)

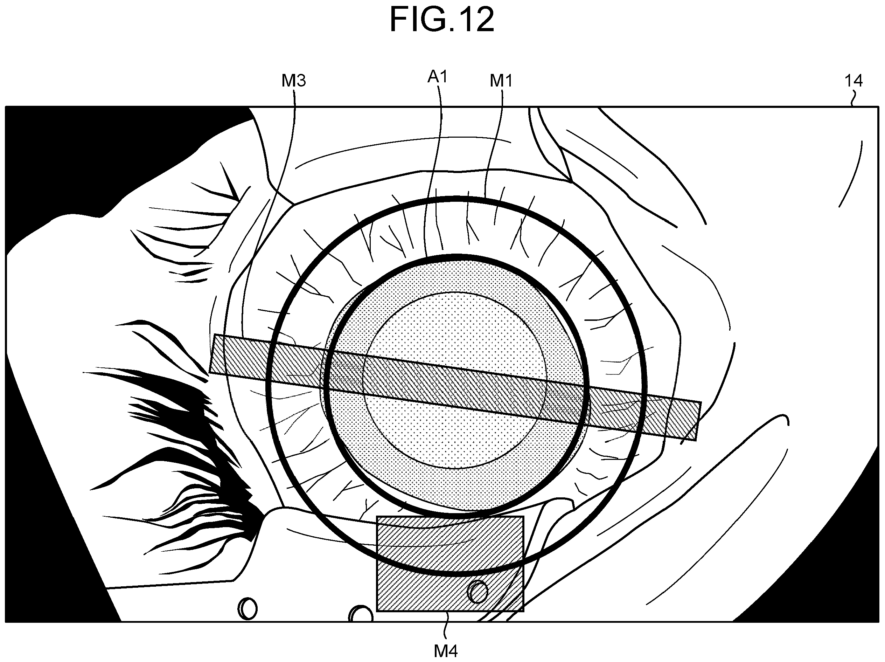

FIG. 12 is a diagram illustrating Example 8 of the display image according to the second embodiment. As illustrated in FIG. 12, a mark M4 is presented to indicate a surgical site planned to be operated after the current surgery. The surgical site is, for example, a glaucoma surgery site. In this manner, in a case where the patient is a glaucoma patient, the mark of the glaucoma surgery site may be superimposed. It should be noted that the mark of the glaucoma surgery site may be a filtering bleb position mark or a mark of the range on the nasal side where many aqueous veins are distributed. Information on the planned surgical site (for example, information such as the position and range of the planned surgical site) is included in preoperative information. The mark M4 corresponds to the second position indicator.

Here, for example, although glaucoma patients may undergo intraocular pressure-lowering surgery such as trabeculectomy, damage to a site such as the filtering bleb before intraocular pressure-lowering surgery should be avoided. In this regard, it is possible to help avoid damage to the site such as the filtering bleb by presenting a filtering bleb position mark. In other words, it is possible to help avoid damage to a planned surgical site by presenting a mark indicating the surgical site planned to be operated after the current surgery. As a similar concept, in performing trabecular meshwork bypass surgery as minimally invasive glaucoma surgery (MIGS), presentation of the range on the nasal side with many aqueous veins important for ensuring improvement in surgical aqueous drainage efficiency is also effective in avoiding damage to the aqueous veins and obtaining a surgical effect. In addition, a mark indicating a past surgical site operated before the current surgery may be presented in addition to the surgical site planned to be operated after the current surgery. In this case as well, it is possible to help avoid damage to the past surgical site.

In addition, each of the marks A1, A1a, M1 to M4, M1a, and the like may be translucent. In addition, each of the marks A1, A1a, M1 to M4, M1a, and the like may be temporarily emphasizable (highlightable) or attenuable (unhighlightable) or may be capable of being repeatedly emphasized and attenuated periodically (the cycle includes one cycle). As a result, it is possible to suppress the marks A1, A1a, M1 to M4, M1a, and the like hindering surgery. In addition, regarding the corneal limbus mark A1, checking of the positional relationship between the corneal limbus and the corneal limbus mark A1 is facilitated.

### (Example 9 of Display Image)

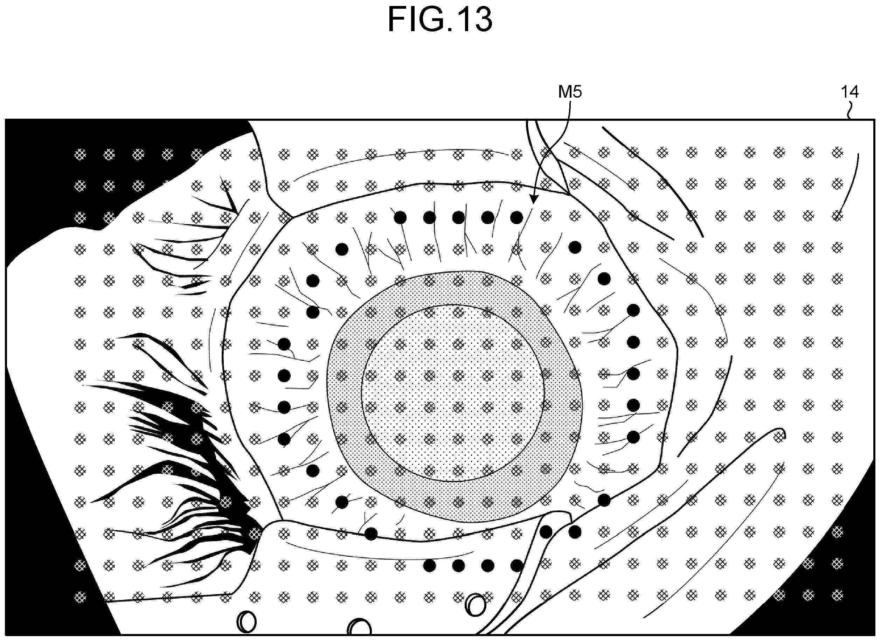

FIG. 13 is a diagram illustrating Example 9 of the display image according to the second embodiment. As illustrated in FIG. 13, a mark M5 is indicated by a display form of a plurality of positionally pre-fixed dots (dot pattern). In the example of FIG. 13, the mark M5 is represented by a plurality of dots (for example, black circles) so as to indicate substantially the same position as the mark M1 illustrated in FIG. 4. Instead of moving each mark A1, A1a, M1, M1a, M2 to M4, and the like in accordance with the eye in the surgical field image, each mark A1, A1a, M1, M1a, and M2 to M4 may be expressed as the mark M5 by changing the color, transmittance, and the like of the positionally pre-fixed dot. As a result, it is possible to perform dot installation interval-based on-screen distance or orientation measurement. It should be noted that details of such a fixed pattern display function will be described later.

Although various display images as described above are used, these display images may be selectable by an operator, a staff member, and the like. The display image selection is made by the operator, staff member, or the like performing an input operation with respect to an operation unit. For example, the operator, staff member, or the like operates the operation unit to select a display mode for displaying a desired display image. In response to this selection, the display image generation unit 13c generates a display image based on the selected display mode. Likewise, regarding the various images, the sizes, positions, and the like of the images may be changeable by the operator, staff member, and the like. The display image generation unit 13c generates a display image by changing, for example, the size and position of the image in response to an input operation that the operator, staff member, or the like performs with respect to the operation unit.

### <2-2. Action and Effect>

As described above, according to the second embodiment, the same effects as those of the first embodiment can be obtained. In addition, the preoperative information receiving unit 13d is further provided to receive preoperative information based on a preoperative plan, and the display image generation unit 13c generates the treatment target position-related first position indicator (for example, marks M1, M1a, and M5) based on the preoperative information to generate a display image including the first position indicator. As a result, it is possible to present the first position indicator based on the preoperative information, and thus the operator can accurately grasp the treatment target position.

In addition, the display image generation unit 13c generates the second position indicator (for example, marks A1, A1a, M2, M3, M4, and M5) different from the treatment target position-related first position indicator based on the preoperative information to generate a display image including the first position indicator and the second position indicator. As a result, the operator can grasp another position to be intraoperatively recognized while grasping the treatment target position. Therefore, the operator does not have to perform measurement on his or her own using an instrument such as a caliper in order to grasp the treatment target position and another position to be recognized, and thus an ophthalmic surgery-related work process can be simplified.

In addition, the first position indicator may be, for example, a curved line or an annular line indicating a position a predetermined distance away from a specific part or a line segment or a point-shaped figure substantially matching a part of the annular line. As a result, it possible to present a plurality of treatment target positions (for example, three port installation positions) on the curved line or annular line, and thus the operator can easily grasp the treatment target position.

In addition, the first position indicator may be a line with a constant width. As a result, the range of the treatment target position is easy to understand, and thus the operator can easily grasp the treatment target position.

In addition, the preoperative information may include information indicating the blood vessel running portion in the eye, and the second position indicator may be an indicator indicating the blood vessel running portion. As a result, the operator can grasp the blood vessel (blood vessel running portion) position and range in detail, and thus it is possible to perform treatment at a position near the blood vessel while avoiding the blood vessel running portion.

In addition, the preoperative information may include information indicating the left-right direction orthogonal to the eye axis of a preoperative image of the eye, and the second position indicator may be an indicator extending in the left-right direction. As a result, the operator can grasp the position and range of a blood vessel, and thus it is possible to perform treatment at a position near the blood vessel while avoiding the blood vessel running portion.

In addition, the preoperative information may include information indicating a predetermined direction predetermined by an operator, and the second position indicator may be an indicator extending in the predetermined direction. As a result, the operator can grasp the position and range of the part predetermined by himself or herself, and thus it is possible to perform treatment at the part or with the part avoided.

In addition, the second position indicator may be an indicator indicating a surgical site planned to be operated after the current surgery or a past surgical site operated before the current surgery. As a result, the operator can grasp the planned surgical site and the past surgical site, and thus it is possible to perform treatment while avoiding the surgical sites.

In addition, one or both of the first position indicator and the second position indicator may be translucent. As a result, it is possible to suppress the presented position indicator hindering surgery.

In addition, one or both of the first position indicator and the second position indicator may be an emphasizable (highlightable) or attenuable (unhighlightable) indicator. As a result, it is possible to highlight the presented indicator or suppress the presented indicator hindering surgery.

In addition, one or both of the first position indicator and the second position indicator may be a repeatedly emphasized (highlighted) and attenuated (unhighlighted) indicator. As a result, it is possible to highlight the presented indicator or suppress the presented indicator hindering surgery.

In addition, one or both of the first position indicator and the second position indicator may be an indicator indicated by a display form of a plurality of positionally fixed dots. As a result, various indicators can be presented with ease.

### <2-3. Example of Fixed Pattern Display Function>

The mark M5 is presented (see FIG. 13) in the display form of the plurality of dots (display image Example 9), and a medical observation system (example of the surgical microscope system 1) with the fixed pattern display function for reference in treatment is implemented so that high-precision treatment is facilitated even in an affected area with few landmarks. Features of the fixed pattern display function will be described.

### (Feature 1)

A plurality of fixed patterns such as dots are displayed in the same shape on an observation screen that is a surgical field image. In addition, the mutual disposition of the fixed patterns is a disposition that is regular in the up-down and left-right directions (also possible is a diagonal lattice rotated by 45 degrees). In addition, the shape and disposition of each fixed pattern do not change during surgery. In addition, the brightness, color, and observation screen transmittance of each fixed pattern change individually. In addition, the brightness, color, and observation screen transmittance of each fixed pattern change only at a part of one fixed pattern.

### (Feature 2)

The fixed patterns are of at least two types in shape. Being of two types in shape means, for example, images such as long and short ruler scales. The disposition of the two types of fixed patterns is also a disposition that is mutually regular in the up-down and left-right directions. The disposition of the two types of fixed patterns (A and B) is an alternate disposition such as "ABABAB...". Alternatively, as in "AAAABAAAABA...", a plurality of As followed by one B is repeated.

### (Feature 3)

The brightness, color, and observation screen transmittance of each fixed pattern do not change in the entire fixed pattern set. Individual fixed patterns that meet a condition change. The condition under which the fixed pattern changes is one of the following. Condition (1) is a case of extracting a feature of an affected area corresponding to a preset feature from an observation image and being within a predetermined range from the extracted feature portion. Condition (2) is a case of being within a preset range.

In (1) above, the preset feature may be associated or unassociated with a preoperative feature of the affected area. The former is the state of the patient's eye obtained from preoperative examination data, such as blood vessel running, pupil ring shape, pupil center, and intra-pupil pattern. As a result, the position of the patient's eye is recognized, and a treatment reference information display position is set in association with the patient's eye. The latter is the color, shape, and size of the subject. As a result, the presentation of the fixed pattern is changed in the event of subject bleeding or depending on the presence or absence of reference color information for fluorescence- or dyeing agent-based treatment.

In (2) above, it is possible to set a part that requires fixed pattern display and a part that does not require fixed pattern display. Simultaneously applied conditions may be a plurality of conditions different in content. The brightness, color, and observation screen transmittance of the fixed pattern changed under different conditions change differently. Alternatively, the change in fixed pattern may be the same even under different conditions.

### (Feature 4)

The interval of the regular disposition of the fixed patterns is set in advance. In addition, two or more types of intervals are selectable in the setting.

### (Feature 5)

The interval of the regular disposition of fixed patterns can be changed in accordance with an observation device state (focus, zoom, setting, arm angle) or a subject feature detection result (one interval being equal division of pupil diameter into n).

### (Feature 6)

The interval of the regular disposition of the fixed patterns varies depending on the part of the observation screen. For example, the interval is close at the middle part of the screen and loose at the peripheral part of the screen or, conversely, the peripheral part of the screen is fixed pattern-less.

### (Feature 7)

The fixed pattern is one and reticulated instead of being plural. Another feature is that "individual fixed pattern" of the above features is replaced with "part of one reticulated fixed pattern".

### (Feature 8)

A plurality of observation screens are simultaneously displayed by PIP/POP, at least one observation screen displays a fixed pattern, and at least one observation screen does not display a fixed pattern. In addition, the interval of fixed pattern display changes in accordance with the screen size of the observation screen.

### (Feature 9)

A plurality of observation screens are simultaneously displayed by PIP/POP, at least one observation screen displays the fixed pattern, and at least one observation screen displays a fixed pattern different therefrom in any of changes in interval, shape, brightness, and the like.

### (Other Features)

The shape of the fixed pattern may be a geometric pattern such as a black circle, a black quadrangle, and a black triangle, a hollow shape such as a white circle, or a radial shape such as a plus "+" and an asterisk "*". By partially changing the fixed pattern, it is possible to make one such as a black circle and an asterisk look like "+", "/", "-", or " | " . In addition, a change in the fixed pattern may be changed in association with the brightness or color of an observation image. For example, changes are possible such as the color opposite to the color of the observation image and a 20% reduction in the brightness of the observation image.

By making the fixed pattern identical in color or brightness to the observation image or considerably increasing the transmittance of the fixed pattern, the fixed pattern can be changed to the extent of being practically unrecognizable visually. In addition, the dimension of the interval of the fixed pattern is displayed on an observation screen. Possible is display in the form of a scale map of a map or the like. The dimension can be calculated from photographing conditions (focus, zoom magnification, observation distance, and the like).

A medical observation device (one example of the surgical microscope 10) is a 3D observation device, and the display of the fixed pattern is 3D or 2D. In a case where the display of the fixed pattern is 2D, the fixed pattern may be displayed on only one of the left and right or the same fixed pattern may be displayed on both the left and right.

A fixed pattern disposition position is defined on a 3D space with respect to a subject. As a result, the interval of the fixed pattern changes in the form of a bird's-eye view in the case of an oblique observation direction. In this case, it is possible to use a 3D space detection function (such as 3D measurement, arm angle detection, and position detection with an external sensor) during fixed pattern creation.

In a case where a feature of the observation image is detected and the fixed pattern is changed, the display range of the fixed pattern indicates the range in which the feature of the observation image can be detected. As a result, an operator can detect the feature of the observation image by capturing an affected area subject to feature detection within the display range of the fixed pattern.

Such medical observation devices are used in surgical operations that require high-precision treatment. However, an affected area is a living tissue and thus has few treatment reference landmarks. For this reason, an operator needs to perform high-precision treatment while contrasting the amount of hand movement with the amount of movement of, for example, a surgical instrument in an observation image. Desirable is display serving as a guideline in an affected area with few landmarks.

In this regard, an observation screen displays a fixed pattern for assisting in the treatment of an affected area. As a result, by the fixed pattern serving as a guideline, an operator's burden during high-precision treatment can be reduced.

### <3. Third Embodiment>

### <3-1. Example of Boundary Display Function Based on Difference in Display Form>

Although a mark is used to indicate a position a predetermined distance away in a specific direction from a specific part of the eyeball in each of the above embodiments, the present invention is not limited thereto and a boundary as a treatment indicator may be used instead of the mark. The boundary is a boundary between a plurality of regions different in display form (such as luminance and saturation). This boundary functions as a position indicator. It should be noted that the difference in display form is a difference in display condition-related parameter, examples of which include differences in luminance, saturation, color temperature, color, contrast, and sharpness. For example, the display form includes a state where a parameter such as the luminance, saturation, color temperature, color, contrast, and sharpness of a surgical field image has changed. In other words, the difference in display form is generated by changing the parameter such as the luminance, saturation, color temperature, color, contrast, and sharpness of the surgical field image.

Here, image processing for generating the boundary with the regions different in display form is implemented by parameter adjustment such as luminance (brightness) adjustment, contrast (shade) adjustment, saturation adjustment, color temperature adjustment, sharpness adjustment, grayscaling, and changing a specific color to another specific color, that is, a change in image pixel value. Specifically, for example, calculation formula-based processing (for example, gain adjustment, offset processing, non-linear operation such as y processing), processing with a lookup table (for example, change from a specific color to a specific color, conversion from a specific luminance value to a specific luminance value for contrast change), processing with a spatial filter, and the like can be used alone or in combination. At this time, the display image generation unit 13c may automatically select and execute boundary-highlighting processing with respect to the original surgical field image (original image). It should be noted that an S-curve change in contrast is an example of the conversion from a specific luminance value to a specific luminance value.

In the image processing, for example, a specific channel is changed regarding the luminance. In addition, for example, a gain is added in accordance with the value of a specific channel regarding the contrast. Regarding the saturation, for example, a uniform gain is added to a specific channel. Regarding the color temperature, a uniform gain different for each channel is added. Regarding the grayscaling, for example, a specific channel value is changed. Regarding the color change, for example, conversion is performed in accordance with a pixel value. It should be noted that in the image generation, an information pattern to be presented or the processing method may be changed based on instructions from a user such as an operator.

Here, the image has, for example, color information in a channel form. An RGB image has the three channels of red, green, and blue. In addition, an HSL image has the three channels of hue, saturation, and luminance (lightness/luminance or intensity). In addition, a CMYK image has the four channels of cyan, magenta, yellow, and black.

In the surgical microscope system 1 configured to use such a boundary, occlusion (shielding) is prevented, an operator can easily see a surgical field image, and the specific position described above can also be grasped by using a display image presenting a specific position based on a preoperative plan with the boundary of the plurality of regions different in display form, and thus ophthalmic surgery following the preoperative plan can be performed with high precision. Various images can be used as the display image for guidance, and a display image including the boundary instead of the mark will be described.

### (Example 10 of Display Image)

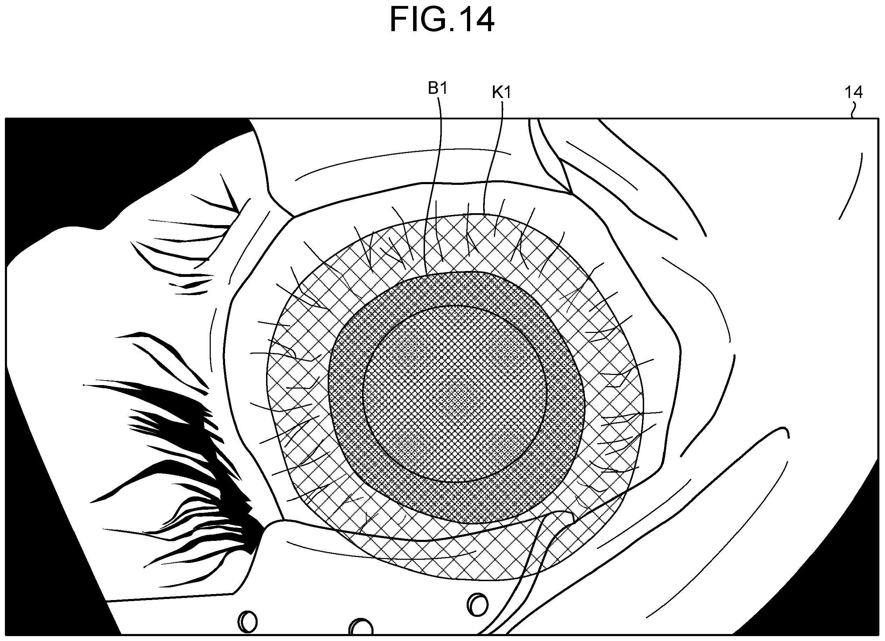

FIG. 14 is a diagram illustrating Example 10 of the display image including boundaries B1 and K1 instead of the marks A1 and M1 illustrated in FIG. 4, respectively. As illustrated in FIG. 14, for example, the luminance of the corneal region is lowered to present the corneal limbus-indicating boundary B1 and, further, the luminance of the region (corneal region-inclusive defined region) defined by the annular position a predetermined distance away from the corneal limbus is lowered to present the annular boundary K1. It should be noted that the luminance of the corneal region is lowered in two stages, and thus the luminance of the corneal region is different from the luminance of the peripheral region (region excluding the corneal region from the defined region) and, for example, the luminance of the corneal region is lower than the luminance of the peripheral region. These boundaries B1 and K1 are included in the real-time surgical field image to generate a display image. This display image is displayed by both or one of the monitor 14 and the presentation unit 55. For example, the boundary K1 indicates a position a certain predetermined distance (for example, approximately 4 mm) away from the corneal limbus in a specific direction from the center of the corneal limbus toward each treatment target point. The boundary K1 corresponds to the first position indicator. In addition, the boundary B1 corresponds to the second position indicator.

According to such a display image, it is possible to obtain the same effect as in the case of using the marks A1 and M1 illustrated in FIG. 4 and, further, it is possible to prevent the occurrence of occlusion (shielding) and perform surgery following a preoperative plan with high precision by presentation with the boundaries between the regions different in display form (such as the boundaries B1 and K1). For example, although occlusion is caused by a mark (such as the marks A1 and M1) overlapping a surgical field image and making a part of the surgical field image invisible, the occurrence of such occlusion can be suppressed by using the boundaries.

### (Example 11 of Display Image)

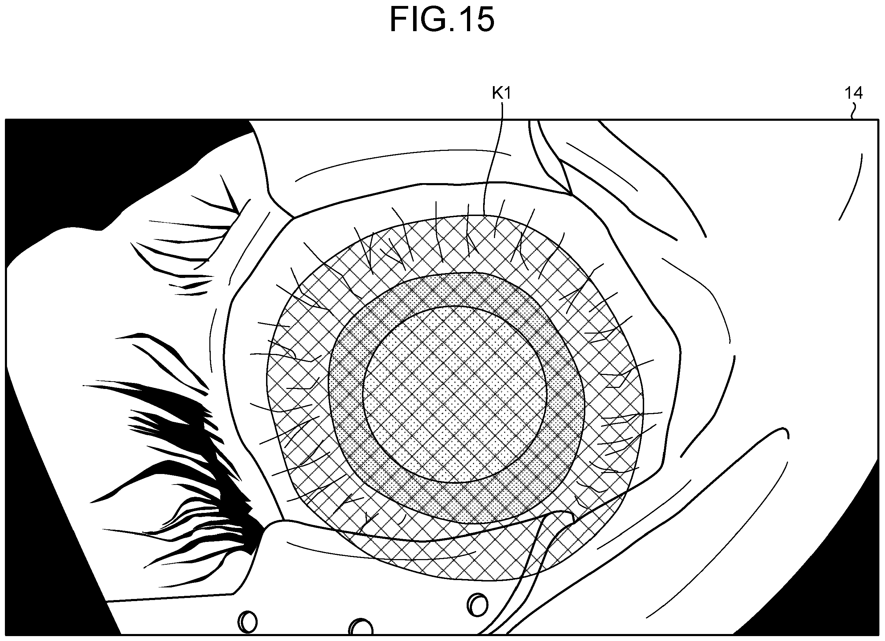

FIG. 15 is a diagram illustrating Example 11 of the display image including the boundary K1 without including the boundary B1 illustrated in FIG. 14. As illustrated in FIG. 15, for example, only the luminance of the region (corneal region-inclusive region) defined by a position a predetermined distance away from the corneal limbus is lowered to present the boundary K1. This boundary K1 is included in the real-time surgical field image to generate a display image.

It should be noted that information can be transmitted if the regions on both sides of the boundary K1 differ from each other (in display form), and thus the intensity of processing with respect to the region (for example, modulation intensity) may be reduced as the distance from the boundary K1 increases. For example, in a case where the processing with respect to the region is processing for an increase in luminance, the luminance of the region decreases as the distance from the boundary K1 increases if the intensity of the processing for increasing the luminance with respect to the region decreases as the distance from the boundary K1 increases. By decreasing the intensity of the processing with respect to the region as the distance from the boundary K1 increases in this manner, it is possible to weaken the difference between the original image and the processed surgical field image at a part away from the boundary K1. In other words, it is possible to maintain the clarity of the gap at the boundary K1 and bring the region away from the boundary K1 closer to the original image.

### (Example 12 of Display Image)

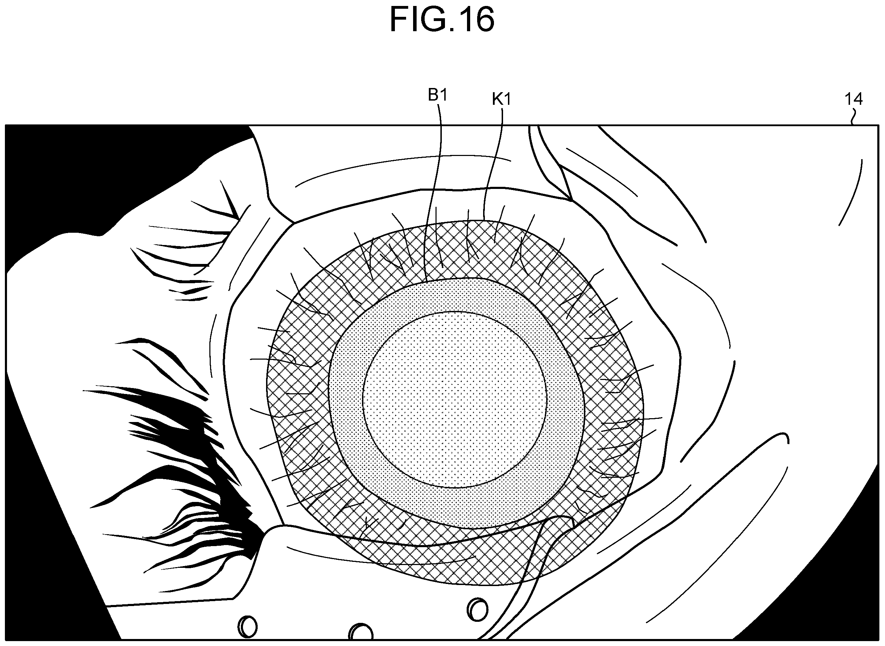

FIG. 16 is a diagram illustrating Example 12 of the display image including the boundaries B1 and K1 illustrated in FIG. 14. As illustrated in FIG. 16, for example, only the luminance of the peripheral region described above is lowered to present each of the boundaries B1 and K1. These boundaries B1 and K1 are included in the real-time surgical field image to generate a display image.

### (Example 13 of Display Image)

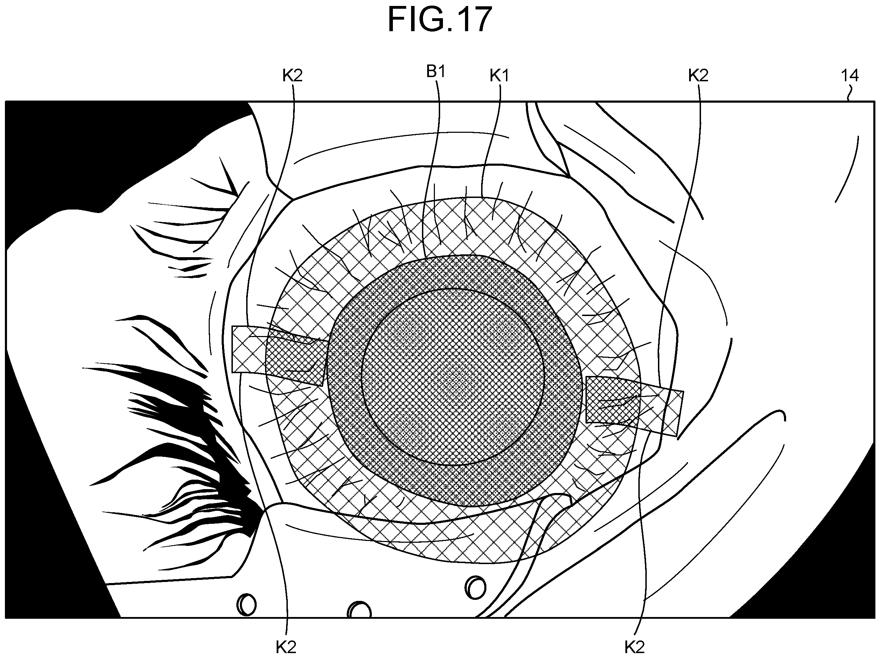

FIG. 17 is a diagram illustrating Example 13 of the display image including each boundary K2 in addition to the boundaries B1 and K1 illustrated in FIG. 14. As illustrated in FIG. 17, for example, the luminance of the region of the blood vessel running portion is lowered in addition to the luminance illustrated in FIG. 14 to present each blood vessel running portion-indicating boundary K2 together with the boundaries B1 and K1. These boundaries B1, K1, and K2 are included in the real-time surgical field image to generate a display image.

### (Example 14 of Display Image)

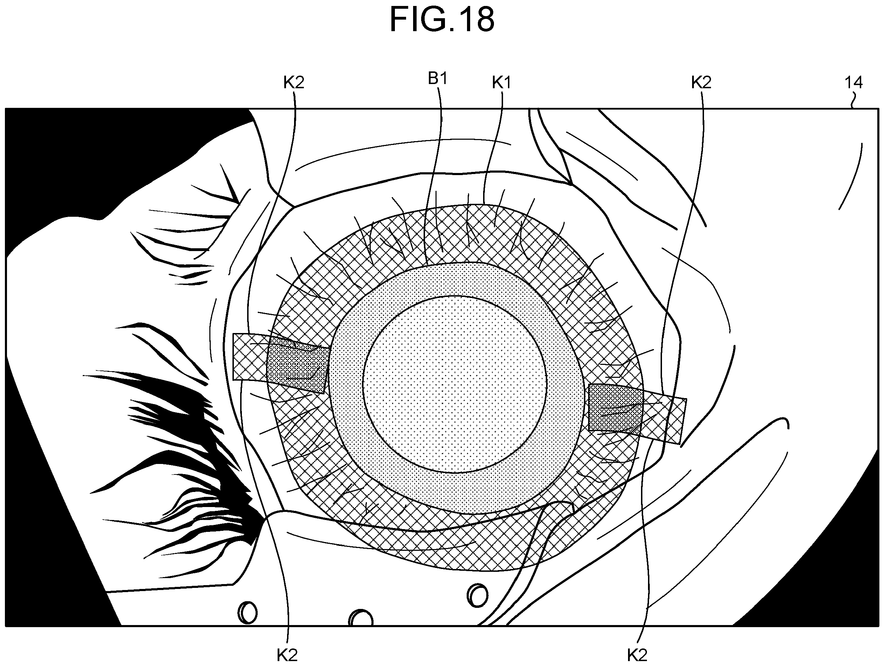

FIG. 18 is a diagram illustrating Example 14 of the display image including the boundaries B1, K1, and K2 illustrated in FIG. 17. As illustrated in FIG. 18, for example, the luminance of the peripheral region described above is lowered to present the boundaries B1 and K1 and, further, the luminance of the region of the blood vessel running portion is lowered to present each blood vessel running portion-indicating boundary K2. These boundaries B1, K1, and K2 are included in the real-time surgical field image to generate a display image.

### (Example 15 of Display Image)

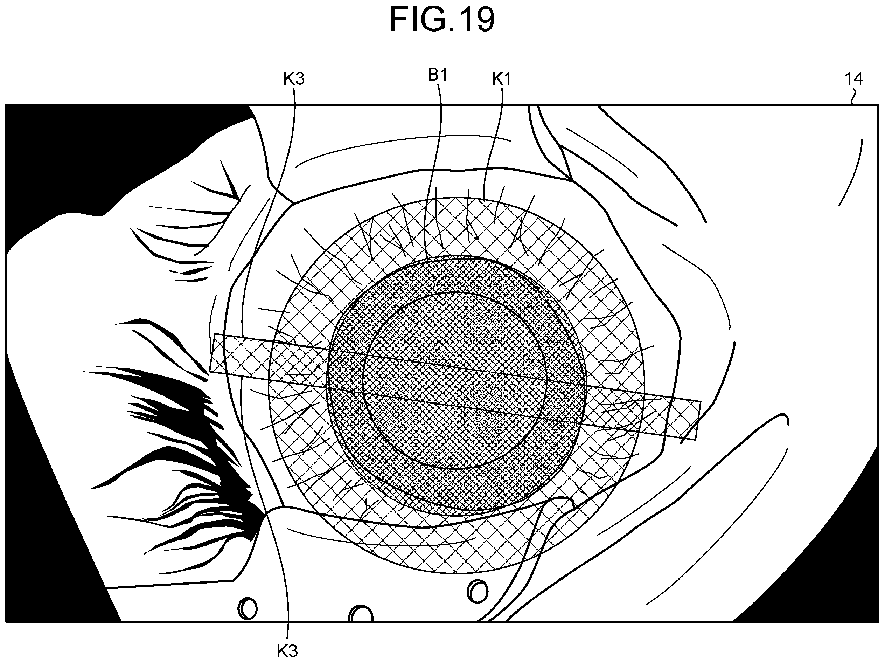

FIG. 19 is a diagram illustrating Example 15 of the display image including the boundaries B1 and K1 and a boundary K3 instead of the marks A1, M1, and M3 illustrated in FIG. 7, respectively. As illustrated in FIG. 19, each of the boundaries B1 and K1 is presented as a perfect circle. For example, the luminance of the region inside the boundary B1 substantially matching the corneal region is lowered to present the boundary B1 and, further, the luminance of the region (corneal region-inclusive defined region) defined by the annular position a predetermined distance away from the boundary B1 is lowered to present the annular boundary K1. Further, the luminance of the band region indicating the 3 o'clock and 9 o'clock directions based on preoperative information is lowered to present each boundary K3. The band region is, for example, a line with a constant width, and the eye is overlaid with the band region. Each of the boundaries B1, K1, and K3 is included in the real-time surgical field image to generate a display image. It should be noted that the orientation of each boundary K3 (position in the direction of rotation around the eye axis) may be changed in accordance with the movement (orientation) of the eyeball so as to be the same at surgery initiation and in real time. The boundary K3 corresponds to the second position indicator.

### (Example 16 of Display Image)

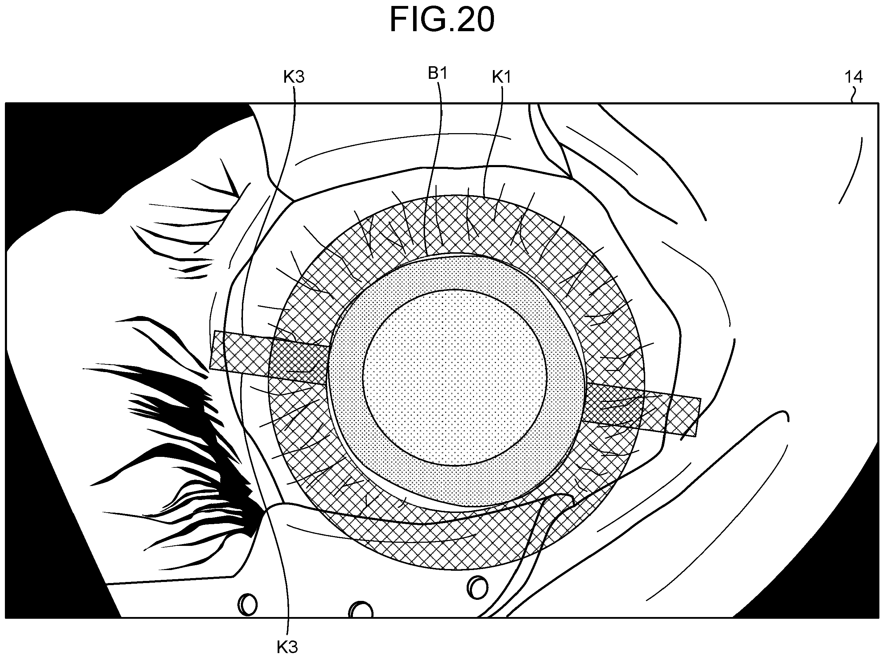

FIG. 20 is a diagram illustrating Example 16 of the display image including the boundaries B1, K1, and K3 illustrated in FIG. 19. As illustrated in FIG. 20, for example, only the luminance of the band region of the perfect circle corresponding to the peripheral region described above is lowered to present each of the boundaries B1 and K1 of the perfect circle. Further, the luminance of the band region indicating the 3 o'clock and 9 o'clock directions based on preoperative information is lowered to present each boundary K3. Each of the boundaries B1, K1, and K3 is included in the real-time surgical field image to generate a display image.

### (Example 17 of Display Image)

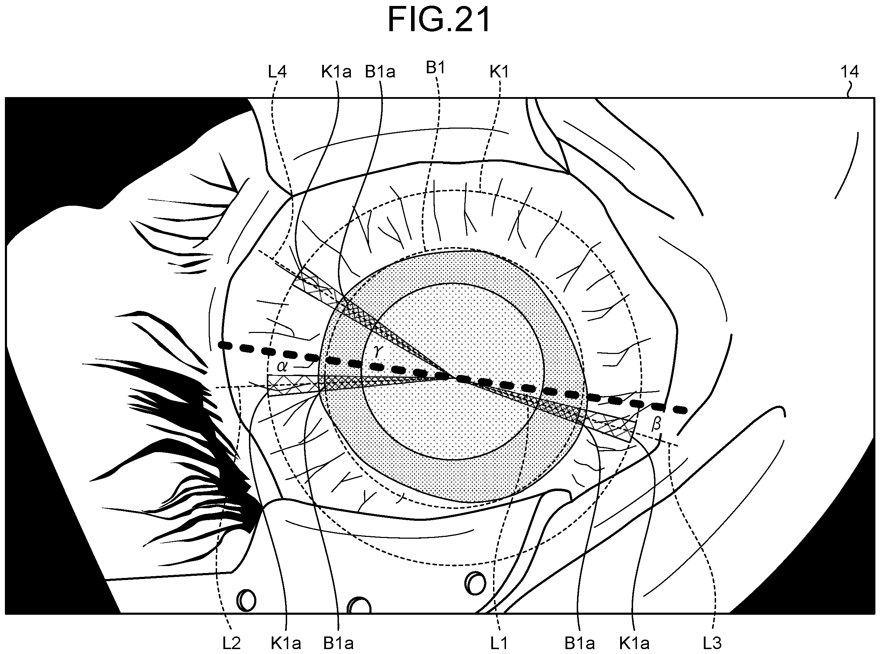

FIG. 21 is a diagram illustrating Example 17 of the display image including each boundary B1 (each partial boundary B1a) and K1 (each partial boundary K1a) instead of each mark A1 (each partial mark A1a) and M1 (each partial mark M1a) illustrated in FIG. 9, respectively. As illustrated in FIG. 21, the boundary B1 of the perfect circle is partially presented as the plurality of partial boundaries B1a. Likewise, the boundary K1 of the perfect circle is partially presented as the plurality of partial boundaries K1a. Each of the partial boundaries B1a and K1a is disposed at angles α, β, and y. For example, at each of the angles α, β, and γ, the luminance of the substantially trapezoidal region between the partial boundary K1a and the partial boundary B1a is lowered and, further, the luminance of the fan-shaped (approximately triangular) region connecting the partial boundary B1a and the center of the boundary B1 of the perfect circle is lowered more than the luminance of the substantially trapezoidal region described above to present each partial boundary K1a and each partial region B1a. Each of the partial boundaries B1a and K1a is included in the real-time surgical field image to generate a display image. The partial boundary K1a corresponds to the first position indicator.

### (Example 18 of Display Image)

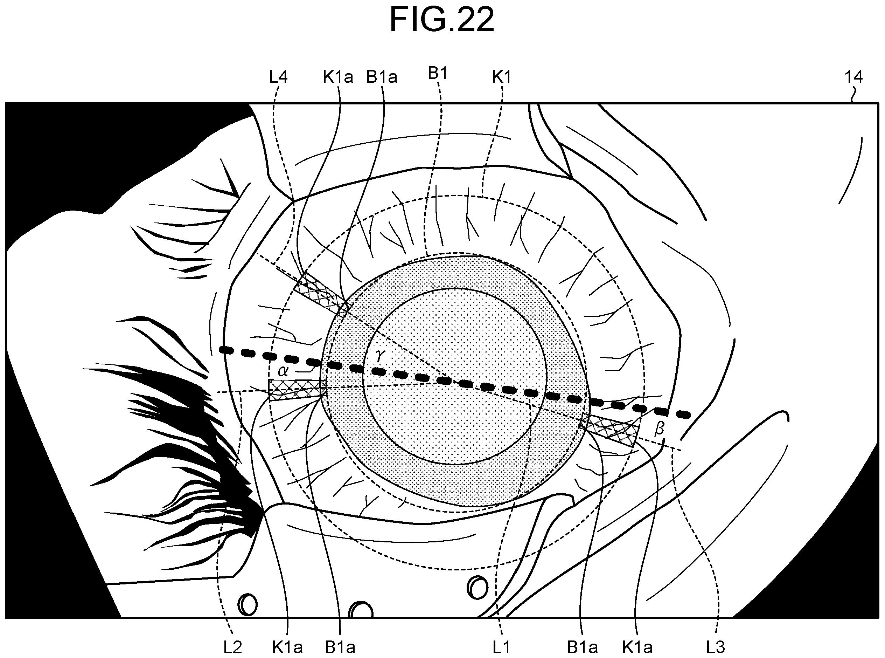

FIG. 22 is a diagram illustrating Example 18 of the display image including each boundary B1 (each partial boundary B1a) and K1 (each partial boundary K1a) illustrated in FIG. 21. As illustrated in FIG. 22, for example, the luminance of the band region of the perfect circle corresponding to the peripheral region described above is partially lowered to present each of the partial boundaries B1a and K1a. Each of the partial boundaries B1a and K1a is included in the real-time surgical field image to generate a display image.

### <3-2. Action and Effect>

As described above, according to the third embodiment, the display image generation unit 13c generates a display image including the boundary (such as the boundaries K1 and K1a) of a plurality of regions different in display form as the first position indicator indicating a position a predetermined distance away from a specific part in a specific direction. As a result, an operator can grasp the position the predetermined distance away in the specific direction from the specific part of the eye, that is, a treatment target position. Therefore, the operator does not have to perform measurement on his or her own using an instrument such as a caliper in order to grasp the treatment target position, and thus an ophthalmic surgery-related work process can be simplified. Further, by presenting the treatment target position with the boundary of the plurality of regions different in display form, occlusion does not occur, the operator can easily see a surgical field image, the treatment target position can also be grasped with reliability, and thus ophthalmic surgery following a preoperative plan can be performed with high precision.

In addition, the boundary of the plurality of regions different in display form (such as the boundaries K1 and K1a) may be the boundary of regions different in luminance. In other words, the difference in display form is generated by changing the luminance of a surgical field image. As a result, occlusion can be suppressed with reliability, and thus an operator can easily see the surgical field image, the treatment target position can also be grasped with reliability, and thus ophthalmic surgery following a preoperative plan can be performed more precisely.

Here, for example, a three-dimensional (3D) image can be used in ophthalmic surgery. In this case, images for the left and right eyes are present such that the sense of depth can be presented as a difference in parallax. Regarding 3D image-related boundary presentation, it is possible to present a boundary (such as the boundaries B1, B1a, K1 to K3, and K1a) on each of the images for the left and right eyes or present a boundary on only one of the images for the left and right eyes. It should be noted that even if a boundary is presented only on an image for one eye, the original image is hardly changed, and thus 3D perception is hardly affected and an operator can have the advantage of being capable of visual boundary recognition. For this reason, the boundary may be presented only on an image for one eye and, in addition, different boundaries may be presented on images for the left and right eyes. For example, different boundaries may be presented on images for the left and right eyes (a first boundary on the image for the right eye and a second boundary on the image for the left eye) and the boundaries may be fused in the brain. In this case, presentable information can be increased by multi-boundary combination.

In addition, the same boundary may be presented on both the image for the left eye and the image for the right eye instead of presenting different boundaries on the images for the left and right eyes. In a case where the same boundary is presented on the images for the left and right eyes, boundary-related depth perception occurs, and thus where to position the boundary may be controlled. For example, processing may be performed so as to create a depth gap with respect to a boundary in a 3D image. As an example of the image processing, the pixels of the images for the left and right eyes are shifted to the right and left, respectively. After the processing (parallax modulation), the parallax changes, and thus the regular position in depth changes and an operator feels as if a boundary or mark popped out in front. As a result, the operator can easily grasp a boundary or mark position, and thus ophthalmic surgery can be performed with high precision.

It should be noted that in a case where the intensity of image processing for boundary formation (such as the degree of modulation) is small, even if only an image of one eye is processed or individual images of both eyes are processed differently, flickering does not occur unlike in mark superimposition and the like and, further, parallax does not occur, and thus surgical field-depth conflict does not occur. In addition, in a case where the same processing is applied to individual images of both eyes, by parallax generation, positioning at a desired specific depth position is possible and a user can be guided for positioning at a treatment position.

In addition, visibility may be improved by periodically reducing the intensity of processing (such as the degree of modulation) of a predetermined region. For example, the processing intensity is gradually reduced from the initial state, gradually increased subsequently, and then returned to the initial state. The visibility can be improved by periodically reducing the intensity of the processing of the predetermined region (degree of image change from the original image) in this manner. In addition, a boundary may be changed in position or size in accordance with a change in eye size.

### <4. Example of Schematic Configuration of Computer>

The above sequential processing can be executed by hardware or software. In a case where the sequential processing is executed by software, a program that configures the software is installed in a computer. Here, examples of the computer include a computer incorporated in dedicated hardware and a general-purpose personal computer capable of executing various functions by installing various programs.

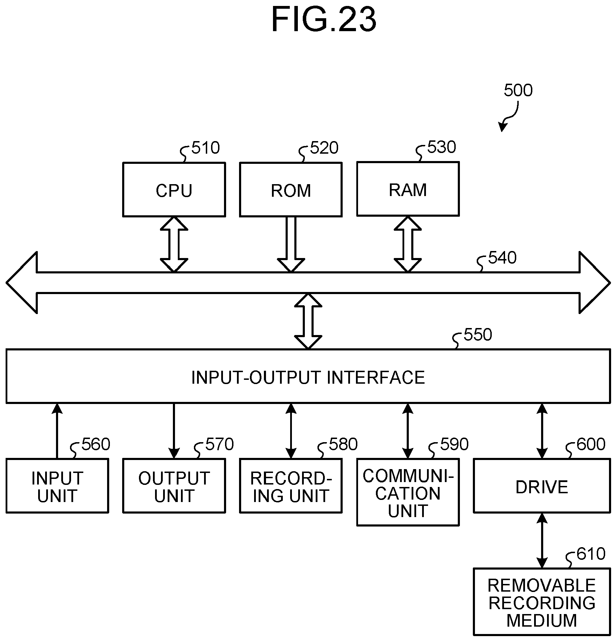

FIG. 23 is a diagram illustrating an example of a schematic configuration of a computer 500 executing the above sequential processing using a program.

As illustrated in FIG. 23, the computer 500 has a central processing unit (CPU) 510, a read only memory (ROM) 520, and a random access memory (RAM) 530.

The CPU 510, the ROM 520, and the RAM 530 are interconnected by a bus 540. Further, an input-output interface 550 is connected to the bus 540. An input unit 560, an output unit 570, a recording unit 580, a communication unit 590, and a drive 600 are connected to the input-output interface 550.

The input unit 560 is configured by a keyboard, a mouse, a microphone, an imaging element, and the like. The output unit 570 is configured by a display, a speaker, and the like. The recording unit 580 is configured by a hard disk, a nonvolatile memory, and the like. The communication unit 590 is configured by a network interface and the like. The drive 600 drives a removable recording medium 610 such as a magnetic disk, an optical disk, a magneto-optical disk, and a semiconductor memory.

In the computer 500 configured as described above, the CPU 510 loads, for example, a program recorded in the recording unit 580 into the RAM 530 via the input-output interface 550 and the bus 540 to execute the program, and the above sequential processing is performed as a result.

The program executed by the computer 500, that is, the CPU 510 can be provided by, for example, being recorded on the removable recording medium 610 such as package media. In addition, the program can be provided via a wired or wireless transmission medium such as a local area network, the Internet, and digital satellite broadcasting.

In the computer 500, the program can be installed in the recording unit 580 via the input-output interface 550 by loading the removable recording medium 610 into the drive 600. In addition, the program can be received by the communication unit 590 and installed in the recording unit 580 via a wired or wireless transmission medium. In addition, the program can be pre-installed in the ROM 520 or the recording unit 580.

It should be noted that the program executed by the computer 500 may be a program in which processing is performed in chronological order in accordance with the order described in this specification or a program in which processing is performed in parallel or at a necessary timing such as when a call is made.

In addition, in this specification, a system means a set of a plurality of components (for example, device, module (parts)), and it does not matter whether every components is in the same housing. Therefore, a plurality of devices stored in separate housings and connected via a network is a system and a single device in which a plurality of modules are stored in one housing is also a system.

In addition, embodiments of the present technology are not limited to the above embodiments, and various modifications are possible without departing from the gist of the present technology.

For example, the present technology is capable of taking a cloud computing configuration in which a single function is shared by a plurality of devices via a network and processed jointly.

In addition, each step described in the above process flow can be executed by a single device or can be shared and executed by a plurality of devices.

Further, in a case where one step includes a plurality of processes, the plurality of processes included in the one step can be executed by one device or shared and executed by a plurality of devices.

In addition, the effects described in this specification are merely examples and are not limited, and there may be effects other than those described in this specification.

### <5. Note>

It should be noted that the present technology can also be configured as follows.
(1) An image processing device comprising:
   an image input unit that receives a surgical field image with respect to a patient's eye;
   a part detection unit that detects a specific part of the eye in the surgical field image; and
   a display image generation unit that generates a display image including a first position indicator indicating a position a predetermined distance away in a specific direction from the specific part detected by the part detection unit.
(2) The image processing device according to (1),
   wherein the first position indicator is a curved line, an annular line, a line segment, or a point-shaped figure indicating the position the predetermined distance away from the specific part.
(3) The image processing device according to (1) or (2),
   wherein the first position indicator is a line with a constant width.
(4) The image processing device according to any one of (1) to (3),
   wherein the first position indicator is translucent.
(5) The image processing device according to any one of (1) to (4),
   wherein the first position indicator is an emphasizable or attenuable indicator.
(6) The image processing device according to any one of (1) to (4),
   wherein the first position indicator is a repeatedly emphasized and attenuated indicator.
(7) The image processing device according to any one of (1) to (6),
   wherein the first position indicator is an indicator indicated by a display form of a plurality of positionally fixed dots.
(8) The image processing device according to any one of (1) to (7),
   wherein the first position indicator is a boundary of a plurality of regions different in display form.
(9) The image processing device according to (8),
   wherein the difference in display form is generated by changing luminance of the surgical field image.
(10) The image processing device according to any one of (1) to (9), further comprising
   a preoperative information receiving unit that receives preoperative information based on a preoperative plan,
   wherein the display image generation unit generates the first position indicator based on the preoperative information and generates the display image including the first position indicator.
(11) The image processing device according to any one of (1) to (10),
   wherein the display image generation unit generates a second position indicator different from the first position indicator and generates the display image including the first position indicator and the second position indicator.
(12) The image processing device according to (11),
   wherein the second position indicator is an indicator indicating a corneal limbus as the specific part.
(13) The image processing device according to (11),
   wherein the second position indicator is an indicator indicating a blood vessel running portion in the eye.
(14) The image processing device according to (11),
   wherein the second position indicator is an indicator extending in a left-right direction orthogonal to an eye axis of a preoperative image of the eye.
(15) The image processing device according to (11),
   wherein the second position indicator is an indicator extending in a predetermined direction predetermined by an operator.
(16) The image processing device according to (11),
   wherein the second position indicator is an indicator indicating a surgical site planned to be operated after current surgery or a past surgical site operated before the current surgery.
(17) The image processing device according to any one of (11) to (16),
   in which the second position indicator is translucent.
(18) The image processing device according to any one of (11) to (17),
   in which the second position indicator is an emphasizable or attenuable indicator.
(19) The image processing device according to any one of (11) to (17),
   in which the second position indicator is a repeatedly emphasized and attenuated indicator.
(20) The image processing device according to any one of (11) to (19),
   in which the second position indicator is an indicator indicated by a display form of a plurality of positionally fixed dots.
(21) The image processing device according to any one of (11) to (20),
   wherein the second position indicator is a boundary of a plurality of regions different in display form.
(22) The image processing device according to (21),
   wherein the difference in display form is generated by changing luminance of the surgical field image.
(23) An image processing method comprising:
   by an image processing device,
   receiving a surgical field image with respect to a patient's eye;
   detecting a specific part of the eye in the surgical field image; and
   generating a display image including a first position indicator indicating a position a predetermined distance away in a specific direction from the specific part.
(24) A surgical microscope system comprising:
   a surgical microscope that obtains a surgical field image with respect to a patient's eye;
   an image processing device that generates a display image; and
   a display device that displays the display image,
   wherein the image processing device includes
   an image input unit that receives the surgical field image,
   a part detection unit that detects a specific part of the eye in the surgical field image, and
   a display image generation unit that generates the display image including a first position indicator indicating a position a predetermined distance away in a specific direction from the specific part detected by the part detection unit.
(25) An image processing method using the image processing device according to any one of (1) to (22).
(26) A surgical microscope system including the image processing device according to any one of (1) to (22).

### Reference Signs List

- 1: SURGICAL MICROSCOPE SYSTEM
- 10: SURGICAL MICROSCOPE
- 11: OBJECTIVE LENS
- 12: EYEPIECE LENS
- 13: IMAGE PROCESSING DEVICE
- 13A: CONTROL UNIT
- 13a: IMAGE INPUT UNIT
- 13b: EYEBALL TRACKING UNIT
- 13c: DISPLAY IMAGE GENERATION UNIT
- 13d: PREOPERATIVE INFORMATION RECEIVING UNIT
- 14: MONITOR
- 20: PATIENT BED
- 51: LIGHT SOURCE
- 52: OBSERVATION OPTICAL SYSTEM
- 52a: HALF MIRROR
- 53: FRONT IMAGE CAPTURING UNIT
- 54: TOMOGRAPHIC IMAGE CAPTURING UNIT
- 55: PRESENTATION UNIT
- 56: INTERFACE UNIT
- 57: SPEAKER
- 500: COMPUTER
- 510: CPU
- 520: ROM
- 530: RAM
- 540: BUS
- 550: INPUT-OUTPUT INTERFACE
- 560: INPUT UNIT
- 570: OUTPUT UNIT
- 580: RECORDING UNIT
- 590: COMMUNICATION UNIT
- 600: DRIVE
- 610: REMOVABLE RECORDING MEDIUM

## Claims

1. An image processing device comprising:
an image input unit that receives a surgical field image with respect to a patient's eye;
a part detection unit that detects a specific part of the eye in the surgical field image; and
a display image generation unit that generates a display image including a first position indicator indicating a position a predetermined distance away in a specific direction from the specific part detected by the part detection unit.

2. The image processing device according to claim 1,
wherein the first position indicator is a curved line, an annular line, a line segment, or a point-shaped figure indicating the position the predetermined distance away from the specific part.

3. The image processing device according to claim 1,
wherein the first position indicator is a line with a constant width.

4. The image processing device according to claim 1,
wherein the first position indicator is translucent.

5. The image processing device according to claim 1,
wherein the first position indicator is an emphasizable or attenuable indicator.

6. The image processing device according to claim 1,
wherein the first position indicator is a repeatedly emphasized and attenuated indicator.

7. The image processing device according to claim 1,
wherein the first position indicator is an indicator indicated by a display form of a plurality of positionally fixed dots.

8. The image processing device according to claim 1,
wherein the first position indicator is a boundary of a plurality of regions different in display form.

9. The image processing device according to claim 8,
wherein the difference in display form is generated by changing luminance of the surgical field image.

10. The image processing device according to claim 1, further comprising
a preoperative information receiving unit that receives preoperative information based on a preoperative plan,
wherein the display image generation unit generates the first position indicator based on the preoperative information and generates the display image including the first position indicator.

11. The image processing device according to claim 1,
wherein the display image generation unit generates a second position indicator different from the first position indicator and generates the display image including the first position indicator and the second position indicator.

12. The image processing device according to claim 11,
wherein the second position indicator is an indicator indicating a corneal limbus as the specific part.

13. The image processing device according to claim 11,
wherein the second position indicator is an indicator indicating a blood vessel running portion in the eye.

14. The image processing device according to claim 11,
wherein the second position indicator is an indicator extending in a left-right direction orthogonal to an eye axis of a preoperative image of the eye.

15. The image processing device according to claim 11,
wherein the second position indicator is an indicator extending in a predetermined direction predetermined by an operator.

16. The image processing device according to claim 11,
wherein the second position indicator is an indicator indicating a surgical site planned to be operated after current surgery or a past surgical site operated before the current surgery.

17. The image processing device according to claim 11,
wherein the second position indicator is a boundary of a plurality of regions different in display form.

18. The image processing device according to claim 17,
wherein the difference in display form is generated by changing luminance of the surgical field image.

19. An image processing method comprising:
by an image processing device,
receiving a surgical field image with respect to a patient's eye;
detecting a specific part of the eye in the surgical field image; and
generating a display image including a first position indicator indicating a position a predetermined distance away in a specific direction from the specific part.

20. A surgical microscope system comprising:
a surgical microscope that obtains a surgical field image with respect to a patient's eye;
an image processing device that generates a display image; and
a display device that displays the display image,
wherein the image processing device includes
an image input unit that receives the surgical field image,
a part detection unit that detects a specific part of the eye in the surgical field image, and
a display image generation unit that generates the display image including a first position indicator indicating a position a predetermined distance away in a specific direction from the specific part detected by the part detection unit.
